# EUROPÄISCHE PATENTANMELDUNG

(11) **EP 0 725 144 A1**
(43) Veröffentlichungstag der Anmeldung: **07.08.1996**
(21) Anmeldenummer: 96101309.1
(22) Anmeldetag: 31.01.1996
(51) Int. Cl.: C12P 19/14, C12P 19/18, C12P 19/20

(54) **Verfahren zur Herstellung von Glycosiden mit Glycosidasen aus Ciliaten**

(30) Priorität: 06.02.1995 DE 19503649
(71) Anmelder: HOECHST AKTIENGESELLSCHAFT, 65929 Frankfurt am Main (DE)
(72) Erfinder: Kiy, Thomas, Dr., D-65929 Frankfurt (DE); Hörsch, Brigitte, Dr., D-65830 Kriftel (DE); Marquardt, Rüdiger, Dr., D-60389 Frankfurt (DE)

(57) **Zusammenfassung**

Die Erfindung betrifft ein Verfahren zur Herstellung von Glycosiden durch enzymatische Glycosylierung mittels α- und β-Glycosidasen aus holotrichen Ciliaten und im besonderen aus Hymenostomatida.

Glycosidasen aus holotrichen Ciliaten eignen sich sehr gut zur enzymatischen Synthese von Alkylglycosiden, Glycopeptiden, Di- bzw. Oligosacchariden. Bevorzugte Glycosyldoren sind Nitrophenylglycoside, Glycosylfluoride oder Disaccharide, bevorzugte Akzeptoren sind kurzkettige Alkohole, Polyhydroxyverbindungen, insbesondere Monosaccharide, oder geschützte Hydroxyaminosäuren oder entsprechende Peptide.

## Beschreibung

Die Erfindung betrifft ein Verfahren zur Herstellung von Glycosiden durch enzymatische Glycosylierung mittels α- und β-Glycosidasen aus holotrichen Ciliaten und im besonderen aus Hymenostomatida.

Kohlenhydrate, deren Glycoside und Glycokonjugate spielen eine zentrale Rolle bei biologischen Erkennungsprozessen (G.E. Edelman, 1984, Spektr. Wiss., 62) wie der Tumorgenese, der bakteriellen und viralen Infektion, der Zell-Zell-Erkennung, dem Zellwachstum und der Zelldifferenzierung (K.G.I. Nilsson, 1988, Trends in Biotechnol., 6, 256). Sie bilden die Grundlagen der Blutgruppenklassifizierung (V. Ginsburg, 1972, Adv. Enzymol., 36, 131) und sind verantwortlich für die Internalisierung verschiedener makromolekularer Stoffe und Medikamente. Ein wichtiges Einsatzgebiet von Glycoproteinen ist z. B. die selektive Adressierung von Pharmaka an das Zielorgan sowie der Schutz von Medikamenten vor proteolytischem Abbau (H. Schachter, 1984, Clinical Biochemistry, 17, 3; N. Sharon, 1984, Trends Biochem. Sci., 9, 198; S. Hakomori, 1985, Cancer Res., 45, 2405; S. Hakomori, 1986, Scientific American, 254, 32). Alkylglycoside können z.B. als Detergenzien oder Emulgatoren eingesetzt werden.

Zentrale Probleme der chemischen Synthese von Glycosiden, Oligosacchariden und Glycokonjugaten sind die stereo- und regioselektive Aneinanderreihung von Monosaccharid-Einheiten, die stereoselektive Knüpfung der glycosidischen Bindung zum Aglycon und nicht zuletzt die Synthese des Aglycons, z. B. eines Peptids.
Die Lösung dieser Probleme erfordert ausgefeilte Synthesekonzepte mit maßgeschneiderten Schutzgruppenkombinationen. Klassisch chemische Synthesen sind daher oft vielstufige, langwierige und häufig nur geringe Mengen der gewünschten Substanz - frei von allen Schutzgruppen- liefernde Reaktionen (H. Kunz, 1987, Angew. Chem., 99, 297; R.R. Schmidt, in "Stereochemistry of Organic and Bioorganic Transformations", W. Bartmann und K.B. Sharpless, Hrsg. VCH Verlagsgesellschaft, Weinheim, S. 169 (1987); H. Waldmann, 1991, Nachr. Chem.Tech. Lab., 39, 675; R.R. Schmidt, 1986, Angew. Chem. 98, 213).

Bei der Synthese von Glycosiden finden zunehmend Enzyme Anwendung. Die damit verbundenen hohen Stereo- und Regioselektivitäten sowie die Vermeidung aufwendiger Schutzgruppenchemie ermöglichen im Vergleich zur vielstufigen chemischen Synthese, häufig gute Ausbeuten. Die bisherigen Ergebnisse auf dem Gebiet der enzymkatalysierten Glycosidsynthese sind folgenden Artikeln zu entnehmen: C.H. Wong et al., 1991, Synthesis, 499; G.M. Whitesides et al., 1989, Tetrahedron, 45, 5362; C. Bucke et al., 1990, Chem. Brit., 657; R.A. Rastall et al., 1992, Biotechn. Gen. Engin. Rev., 10, 253; K.G.I. Nilsson, 1991, Appl. Biocatalysis 1, 117; K.G.I. Nilsson, 1988, Tibtech, 256.

Hierbei sind Glycosidasen wie α- und β-Glucosidasen, α- und β-Galactosidasen, α- und β-Fucosidasen und α- und β-Mannosidasen zur Synthese von Glycosiden eingesetzt worden.

Diese Glycosidasen sind aus Bakterien und Pilzen (z. B. β-Galactosidase aus Escherichia coli, Aspergillus niger, Diplococcus pneumoniae), Pflanzen (z. B. β-Glucosidase aus Mandeln), Tieren (β-Galactosidase aus Rinderleber) und menschlichem Gewebe (α-Fucosidase aus humaner Plazenta) zugänglich. Sie liegen jedoch häufig nur intrazellulär vor und müssen nach Zellaufschluß in zum Teil sehr aufwendigen Chromatographieschritten gereinigt werden.
Viele der Enzyme können daher nur in kleinen Mengen isoliert werden. Bei nicht hochgereinigten Enzymen sind in nicht unerheblichem Maß Nebenaktivitäten vorhanden.

β-Hexosaminidasen, β-N-Acetylglucosaminidasen und β-N-Acetylgalactosaminidasen wurden bisher nur in sehr geringem Ausmaß zur enzymkatalysierten Glycosidsynthese verwendet, insbesondere deshalb, weil diese Enzyme nur schwer in großen Mengen bereitgestellt werden können. (D.H.G. Crout et al., 1992, J. Chem. Soc. Chem. Commun., 704; D.H.G. Crout et al., 1992, Pure & Appl. Chem., 8, 1079; K.G.I. Nilsson, 1990, Carbohyr. Res., 204, 79; K.G.I. Nilsson 1989, Carbohydr. Res., 188, 9).

Es ist daher wünschenswert, leicht zugängliche Glycosidasen mit breitem Substratspektrum und hoher Stabilität für die enzymatische Glycosidsynthese zur Verfügung zu stellen.

Es ist seit langem bekannt, daß das holotriche Ciliat Tetrahymena pyriformis lysosomale Enzyme wie α- und β-Glucosidase, β-N-Acetylglucosaminidase, β-N-Acetylhexosaminidase (β-Hexosaminidase), Amylase, α-Mannosidase, β-Galactosidase und β-Fucosidase in das Medium ausscheidet (M. Müller, 1972, J. Cell Biol., 52, 478; J. J. Blum, 1976, J. Cell Physiol., 89, 457). Im Fall von T. thermophila wurde die Sekretion von β-N-Acetylhexosaminidase, α-Mannosidase, α- und β-Glucosidase beobachtet, wobei die β-N-Acetylhexosaminidase sogar partiell gereinigt und charakterisiert wurde (A. Tiedtke, 1983, Comp. Biochem. Physiol., 75B, 239).

Ferner sind Verfahren zur Gewinnung von β-Hexosaminidase, α-Glucosidase, β-Glucosidase und α-Mannosidase aus T. thermophila-Kulturen publiziert (T. Kiy & A. Tiedtke, 1991, Appl. Microbiol. Biotechnol., 35, 14; T. Kiy & A. Tiedtke, 1993, BioEng., 5, 22).

Bisher wurde jedoch nur die hydrolytische Aktivität der Ciliaten-Glycosidasen z.B. anhand von p-Nitrophenylglycosiden wie p-Nitrophenyl-N-acetyl-β-D-glucosaminid und p-Nitrophenyl-N-acetyl-β-D-galactosaminid im Rahmen der üblichen Enzymtests für Hydrolasen nachgewiesen.

Demgegenüber betrifft die vorliegende Erfindung ein Verfahren zur Herstellung von Glycosiden durch enzymatische Glycosidierung von Glycosylakzeptoren mittels Glycosidasen, welches sich dadurch auszeichnet, daß Glycosidasen aus Ciliaten der Unterklasse Holotrichia, insbesondere der Ordnung Hymenostomatida eingesetzt werden.

Vorzugsweise werden Glycosidasen aus Hymenostomatiden der Gattungen Colpidium, Tetrahymena, Pseudocohnilembus oder Potomacus eingesetzt.

Die Glycosidasen werden von den holotrichen Ciliaten ins Kulturmedium ausgeschleust und sind größtenteils selbst bei Raumtemperatur und ohne Zusatz von Proteaseinhibitoren über lange Zeit stabil. Die Enzyme lassen sich ohne Zellaufschluß aus dem Kulturmedium in großen Mengen gewinnen.

Lysosomale Glycosidasen aus holotrichen Ciliaten weisen überraschend ein breites Substratspektrum für die enzymatische Synthese von Glycosiden auf. Die Glycosidasen aus holotrichen Ciliaten eignen sich insbesondere sehr gut zur enzymatischen Synthese von Alkylglycosiden, Glycopeptiden, Di- bzw. Oligosacchariden. Erstmals wurde somit gezeigt, daß sich lysosomale Hydrolasen aus holotrichen Ciliaten zur Synthese oder zur Derivatisierung von Glycosiden einsetzen lassen.

Als Quelle der Glycosidasen lassen sich sämtliche Vertreter der holotrichen Ciliaten, im besonderen der hymenostomatiden Ciliaten, vorzugsweise Tetrahymeniden, Colpidium, Pseudocohnilembus oder Potomacus heranziehen. So konnten die entsprechenden Enzymaktivitäten in Kulturen von T. thermophila, T. pyriformis, T. pigmentosa, T. specialis und Colpidium campylum, Pseudocohnilembus marinus und Potomacus pottsi nachgewiesen werden (Tab. 1).

Außerdem können auch diverse Stämme und Mutanten verwendet werden, soweit sie die Glycosidasen synthetisieren. Solche Mutanten lassen sich nach der von Hünseler et al. (1987, J. Cell Sci., 88, 47) beschriebenen Methode unter Verwendung von N-Methyl-N'- nitro-N'-nitrosoguanidin gewinnen.

Tabelle 1 stellt eine Übersicht über die Glycosidaseaktivitäten dar, welche in hymenostomatiden Ciliaten nachgewiesen wurden und welche sich gemäß der vorliegenden Erfindung zur enzymatischen Glycosidierung nutzen lassen.

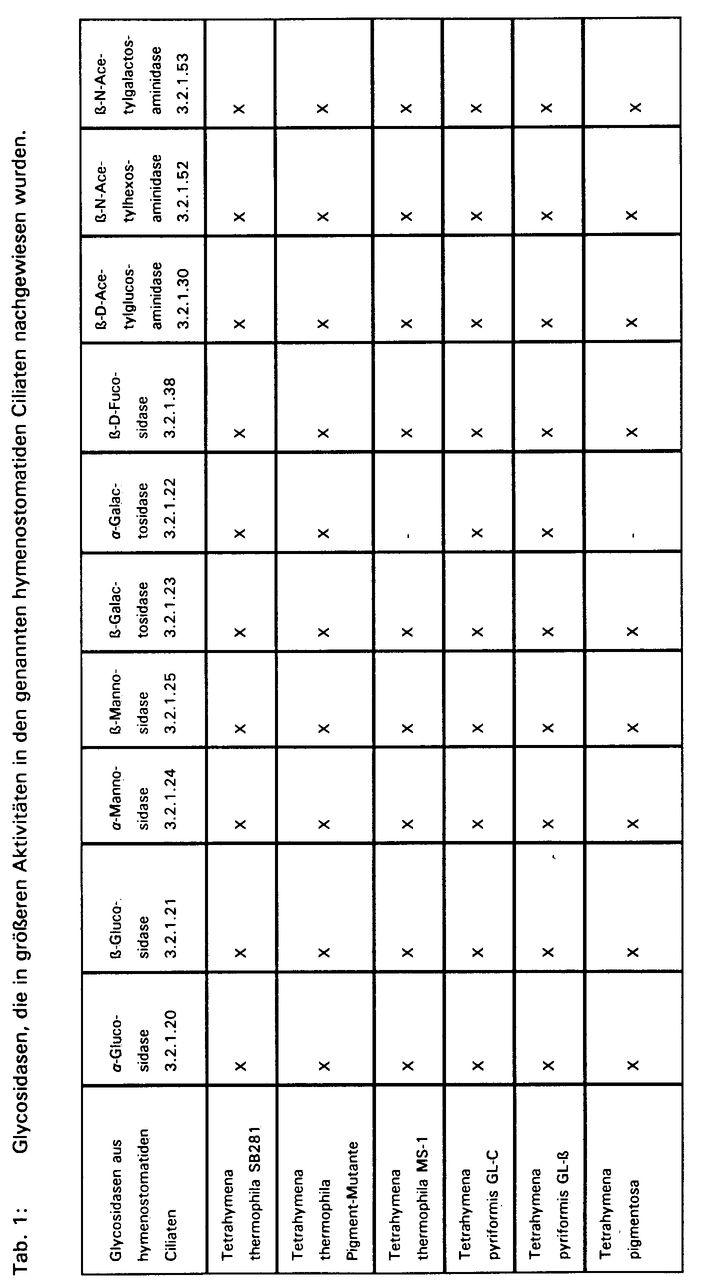

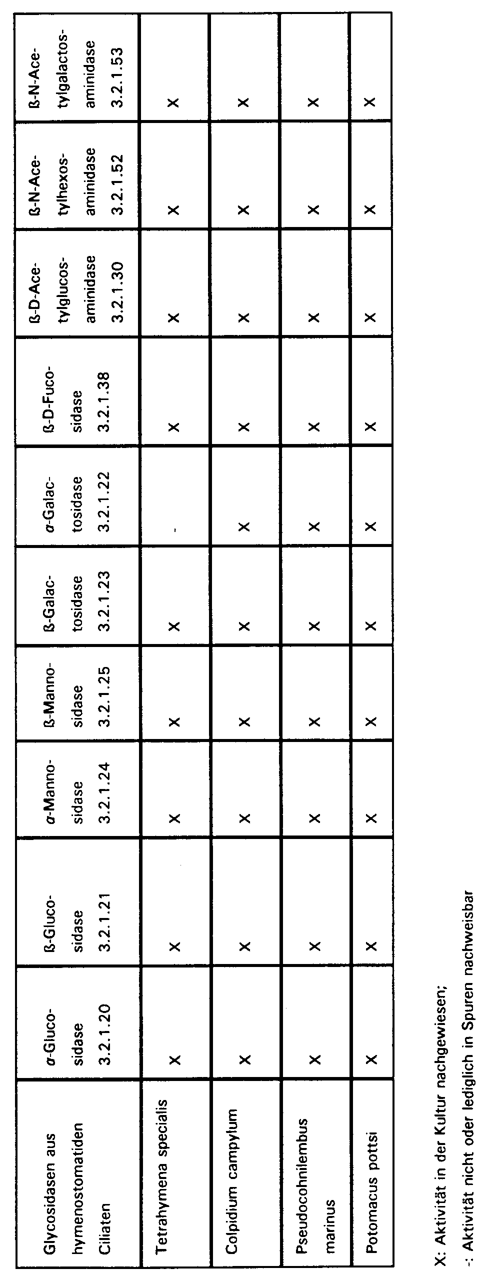

Vorzugsweise werden die Glycosidasen aus dem enzymhaltigen, zellfreien Überstand einer Kultur isoliert. Wahlweise können die Glycosidasen auch aus den Zellen gewonnen werden, wobei diese dann zusätzlich aufgeschlossen werden müssen.

Sollen die Glycosidasen aus dem zellfreien Kulturüberstand isoliert werden, so müssen die Zellen vom Kulturmedium abgetrennt werden. Dies kann z. B. über Zellimmobilisierung (T. Kiy & A. Tiedtke, 1991, Appl. Microbiol. Biotechnol., 35, 18) oder die Verwendung von Membran- bzw. Spinfilterbioreaktoren bereits während der Fermentation geschehen (T. Kiy & A. Tiedtke, 1992, Appl. Microbiol. Biotechnol., 38, 141) oder aber über Tangentialfiltration oder Zentrifugation nach Beendigung der Fermentation.

Sollen zusätzlich die zellassoziierten Glycosidasen gewonnen werden, müssen die Zellen zunächst homogenisiert werden. Besonders geeignet sind in diesem Zusammenhang Ultraschall- bzw. Ultraturraxgeräte, aber auch einfaches Einfrieren reicht aus, um die Zellen aufzuschließen. Über Zentrifugation oder Filtration läßt sich partikuläres Material nach dem Homogenisieren vom Überstand abtrennen.

Die Trennung und Reinigung der Glycosidasen erfolgt über chromatographische Methoden. Wahlweise kann vor der Chromatographie eine Ammoniumsulfatfällung durchgeführt werden. Eine Ammoniumsulfatsättigung von 70% erwies sich hierbei als geeignet, da bei dieser Konzentration nahezu 100% der Aktivitäten von z. B. β-N-Acetylhexosaminidase, α-Mannosidase, α-und β-Glucosidase, β-D-Acetylglucosaminidase und β-N-Acetylgalactosaminidase ausgefällt werden können. Die enzymhaltige Probe läßt sich z. B. zunächst auf eine Anionenaustauschersäule (z. B. Q-Sepharose®, Mono Q®) auftragen. Die Elution erfolgt über einen aufsteigenden Salzgradienten. Alternativ kann eine Chromatofokussiersäule (z. B. Mono P®) eingesetzt werden, wobei mittels eines absteigenden pH-Gradienten eluiert wird. Zur Trennung der Glycosidasen von kleinmolekularen Substanzen hat sich der Einsatz einer Gelfiltration (z. B. Superose®, Superdex®) als sinnvoll erwiesen. Zur gezielten Aufreinigung und Trennung der Glycosidasen voneinander bietet sich der Einsatz von affinitätschromatographischen Medien an.

Folgende Glycosidasen können z.B. aus holotrichen Ciliaten gewonnen werden: β-Glucosidase, β-Galactosidase, β-Mannosidase, β-D-Fucosidase, β-Hexosaminidase, β-N-Acetylglucosaminidase, β-N-Acetylgalactosaminidase, α-Glucosidase, α-Galactosidase, α-Mannosidase, α-L-Fucosidase (s. Tab. 1).

Die ungereinigten oder partiell bzw. vollständig gereinigten Glycosidasen werden zur Synthese von Glycosiden eingesetzt. Hierbei wird ein Glycosyldonor mit einem Acceptor in einer wäßrigen Pufferlösung in Gegenwart des Enzyms zum Glycosid umgesetzt.

Als Glycosyldonoren werden vorzugsweise Nitrophenylglycoside eingesetzt, wie z.B. Mono- oder Dinitrophenyl-β-D-glucosid, Mono- oder Dinitrophenyl-β-D-galactosid, Mono- oder Dinitrophenyl-β-D-mannosid, Mono- oder Dinitrophenyl-β-D-fucoside,Mono- oder Dinitrophenyl-N-Acetyl-β-D-glucosaminide, Mono- oder Dinitrophenyl-N-Acetyl-β-D-galactosaminid, Mono- oder Dinitrophenyl-α-D-glucosid, Mono- oder Dinitrophenyl-α-D-galactosid, Mono- oder Dinitrophenyl-α-D-mannosid, Mono- oder Dinitrophenyl-α-L-fucoside.

Vorzugsweise werden auch Glycosylfluoride wie β-D-Glucosylfluorid, β-D-Galactosylfluorid, β-D-Mannosylfluorid, β-D-Fucosylfluorid, N-Acetyl-β-D-glucosylfluorid und N-Acetyl-β-D-galactosylfluorid, α-D-Glucosylfluorid, α-D-Galactosylfluorid, α-D-Mannosylfluorid, α-L-Fucosylfluorid als Glycosyldonoren eingesetzt.

Als Glycosyldonoren eignen sich ferner auch Disaccharide wie Lactose oder Chitobiose.

Bei den eingesetzten Glycosylakzeptoren handelt es sich vorzugsweise um Alkohole mit 1 bis 10 Kohlenstoffatomen oder um Polyhydroxyverbindungen mit 2 bis 10 Kohlenstoffatomen, insbesondere um
kurzkettige, acyclische oder cyclische, gesättigte oder ungesättigte Mono-, Di-, Tri- oder Polyhydroxyverbindungen mit 1 bis 10 C-Atomen, die wahlweise mit Halogen (F, Cl, Br, I) oder Sulfonyl-, Cyano-, Alkyl-, Alkyloxy-, Aryl-, Aryloxy-, Heteroaryl-,Heteroaryloxy-, Thioalkyl-, Trialkylsilyl-,Trialkylsiloxy-, Azido-Amino-, N-Acylamino-, N-tert-Butyloxycarbonylamino-, N-Benzyloxycarbonylamino-, N-Allyloxycarbonylamino-, N-Methoxycarbonylamino-,N-Phthalimido-gruppen substituiert sind.

Als Glycosylakzeptoren werden vorzugsweise auch Monosaccharide eingesetzt, wie beispielsweise Galactose, Glucose, Mannose, Fucose, Xylose, Lyxose, 2-Deoxy-2-Amino-Zucker oder 2-Deoxy-2-N-Acyl-Zucker, wie beispielsweise N-Acetylglucosamin, oder Glycosylfluoride sowie Alkyl- und Arylglycoside mit Alkyl- bzw. Arylresten mit 1 bis 10 C-Atomen, die wahlweise substituiert sein können mit Halogen, Trialkylsilyl-, Cyano-, Nitro- und Azidogruppen.

Als Glycosylakzeptor eignet sich vorzugsweise eine Verbindung der Formel I 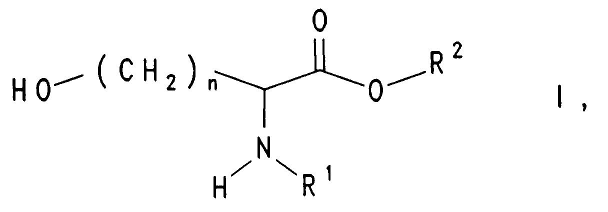 worin bedeuten
- n: eine ganze Zahl von 1 bis 10,
- R¹: eine Aminoschutzgruppe,
- R²: eine Hydroxylgruppe, eine Alkoxyalkylgruppe, Thioalkylgruppe oder eine Alkenyloxygruppe mit jeweils 1 bis 18 Kohlenstoffatomen, die mit Halogen oder Cyan substituiert sein können, oder eine Aryloxygruppe mit 6 bis 10 Kohlenstoffatomen, die mit Alkyl-, Alkoxy-, Thioalkyl-, jeweils mit 1 bis 5 C-Atomen, und Nitrogruppen substituiert sein kann.

Als Schutzgruppe in R¹-Stellung der Formel I können im wesentlichen die in der Peptid- und Glycopeptidchemie gängigen Aminoschutzgruppen eingesetzt werden (T.W. Greene, Protective Groups in Organic Synthesis, John Wiley & Sons 1981, Houben-Weyl Band 15-1, S. 46)

Die verwendeten Glycosylacceptoren und Glycosyldonoren können im Verhältnis 10:1 bis 1:4, bevorzugt 2:3 bis 4:1 eingesetzt werden.

Die Reaktion kann in einem pH-Bereich von 4.5 bis 8.0, vorteilhaft jedoch zwischen pH 5.0 und 7.5 ablaufen.

Die Reaktionstemperatur liegt zwischen 15°C und 50°C, vorzugsweise zwischen 20°C und 35°C.

Die Reaktionszeit beträgt zwischen 30 Minuten und 200 Stunden.

Zur Verbesserung der Löslichkeit der Substrate können Lösungsmittel verwendet werden, die das Enzym in seiner Aktivität nicht oder nur geringfügig negativ beeinflussen. Dies sind z. B. Aceton, Dimethoxyethan, Diglyme, Toluol, Dimethylformamid, Dimethylsulfoxid und Acetonitril.

Der Verlauf der Reaktion kann mit Hilfe der HPLC oder durch DC verfolgt werden. Die anschließende Aufarbeitung erfolgt beispielsweise durch Extraktion mit Diethylether, Gefriertrocknung der wäßrigen Phase und Reinigung durch Säulenchromatographie an Kieselgel oder durch Gelfiltration an Sephadex® oder Biogel®.

### Beispiel 1

Stellvertretend für sämtliche holotrichen Ciliaten sei hier die Kultivierung von T. thermophila sowie die Isolierung und Reinigung der Glycosidasen beschrieben.

### 1. Stammkultur des Produzentenstammes (T. thermophila)

Die Dauerkultivierung erfolgt bei 20°C in Reagenzgläsern auf autoklavierten Kichererbsen. Dazu wird eine Kichererbse in 10 ml Aqua demin. pro Reagenzglas eingesetzt. Das Umimpfen erfolgt halbjährlich.

### 2. Vor- bzw. Hauptkultur des Produzentenstammes in Erlenmeyerkolben

Ein 100-ml-Erlenmeyerkolben, der 10 ml Kulturmedium enthält, wird mit 1 ml der Stammkultur angeimpft und bei 30°C über Nacht auf einem Schüttler (100 rpm) inkubiert. Am nächsten Tag wird ein 500-ml-Erlenmeyerkolben, der 100 ml Kulturmedium enthält, mit 1 ml dieser Vorkultur inokuliert. Nach 24 h werden 500 ml Medium (in 2,5-l-Erlenmeyerkolben) mit 10 ml dieser Vorkultur angeimpft. Die maximale volumetrische Aktivität ist zwischen 40 und 90 h erreicht. Die Kultur enthält z. B. bis zu 800 mU/ml β-Hexosaminidase. Wird die Kultivierung kontinuierlich mit Zellrückhaltung - z. B. in einem Membranbioreaktor - durchgeführt, so ist eine Steigerung der volumetrischen Aktivität bis um das 50fache realisierbar.

Das Produktionsmedium enthält Magermilchpulver (20 g/l), Hefeextrakt (5 g/l), Sequestrene® (0,003%) und Glucose (10 g/l). Die Glucose wird getrennt autoklaviert und erst nach dem Abkühlen dem Kulturmedium zugeführt. Alternativ kann PPYS-Medium® oder Pharmamedia®-Medium ( 20 g/l Pharmamedia®, 10 g/l Glucose, 5 g/l Hefeextrakt und 0,003% Sequestrene oder 1ml/l Eisenspur) verwendet werden.

### 3. Ernte des enzymhaltigen Mediums

Zur Abtrennung der Zellen vom enzymhaltigen Überstand werden 5 l (10 x 500 ml) einer Stationärphasekultur von T. thermophila über eine FILTRON®-Tangentialfiltrationseinheit (Porengröße, 0,3µm) aufgearbeitet. Durch Verwendung einer weiteren Membran (Ausschlußgröße, 10 kDa) wird der zellfreie Überstand bis auf ein Volumen von 300 ml eingeengt. Mittels einer AMICON®-Ultrafiltrationszelle (PM 10-Membran) wird dieses Konzentrat weiter bis auf 30 ml eingeengt und gleichzeitig in den Startpuffer (20 mM Triethanolamin, pH: 7,8) für den ersten Chromatographieschritt umdialysiert. Alternativ können die Glycosidasen vor der Chromatographie aus dem Konzentrat gefällt werden.

### 4. Ammoniumsulfatfällung

Zur Ankonzentrierung der sezernierten Glycosidasen hat sich eine Fällung mit Ammoniumsulfat (70%ige Sättigung) als sinnvoll herausgestellt. Im resuspendierten Präzipitat werden z. B. 95% β-N-Acetylhexosaminidase, 99% β-N-Acetylgalactosaminidase, 100% α-Glucosidase und 93% β-Glucosidase wiedergefunden.,

### 5. Ionenaustauschchromatographie

Die Probe wird auf eine Q-Sepharose®-Säule (26/10) aufgebracht. Nachdem alle ungebundenen Proteine mit Startpuffer von der Säule gewaschen wurden, wird die Elution (aufsteigender Salzgradient, 20 mM Triethanolamin, 1 M NaCl, pH: 7,8) gestartet. Unter diesen Umständen erscheinen einige Glycosidasen, z. B. β-Galactosidase und β-Fucosidase im Durchbruch, während andere Enzyme, z. B. β-N-Acetylhexosaminidase und β-N-Acetylglucosaminidase erst im Verlauf des Gradienten mit ansteigender NaCl-Konzentration eluieren. Die entsprechenden Fraktionen werden gepoolt und über eine AMICON®-Ultrafiltrationszelle in Citratpuffer (50 mM Citrat, 100 mM NaCl, pH: 6,5) umdialysiert.

### 6. Gelfiltration

Dieser Reinigungsschritt wird mittels einer Superose® 12-Säule durchgeführt (Abb. 2). Als Laufpuffer wird Citratpuffer (50 mM Citrat, 100 mM NaCl, pH: 6,5) verwendet. Die enzymatisch aktiven Fraktionen werden erneut gepoolt und über eine AMICON®-Ultrafiltrationszelle in Phosphatpuffer (20 mM Na-Phosphat, 10 mM NaCl, pH: 6,0) umdialysiert.

### 7. Affinitätschromatographie

Zum Aufbringen der Probe wird oben genannter Phosphatpuffer verwendet. Die Elution erfolgt mit einem Tris/HCl-Puffer (20 mM Tris, 150 mM NaCl, pH: 8,5). Als Substratanalogon dient p-Aminophenyl-2-acetamido-2-deoxy-β-D-glucopyranosid, gekoppelt an CNBr-aktivierte Sepharose. Im Durchbruch erscheint dabei die β-Hexosaminidase, während im Verlauf des Gradienten die β-N-Acetylglucosaminidase eluiert. Um auszuschließen, daß dieses Elutionsverhalten etwa nur auf einen Überladungseffekt der Säule zurückzuführen ist, wurde der Durchbruch gepoolt, erneut in Startpuffer umdialysiert und erneut auf die Säule aufgetragen. Wieder erschien die Gesamtaktivität im Durchbruch, so daß davon auszugehen ist, daß sich tatsächlich 2 verschiedene Enzyme, die beide p-Nitrophenyl-N-acetyl-β-D-glucosamin als Substrat erkennen, hinter der β-Hexosaminidase verbergen.

Damit konnte gezeigt werden, daß sich die β-Hexosaminidase aus T. thermophila nochmals in zwei verschiedene Enzymaktivitäten auftrennen läßt. Während eine Fraktion sowohl p-Nitrophenyl-N-acetyl-β-D-glucosaminid als auch p-Nitrophenyl-β-D-galactosaminid hydrolysiert - also eine β-Hexosaminidase ist -, akzeptiert die zweite Form im wesentlichen nur das erst genannte Substrat und ist demnach eine β-N-Acetylglucosaminidase.

### Beispiel 2:

50 mg (0.15 mmol) p-Nitrophenyl-β-D-N-Acetylglucosaminid und 2 mL Methanol werden mit 1 Unit β-Hexosaminidase (Rohenzym) aus T. thermophila in 10 ml 0.1M Citrat-Puffer pH 6.5 19h bei Raumtemperatur gerührt. Nach Extraktion mit Diethylether wird die wäßrige Phase gefriergetrocknet. Nach Chromatographie an Biogel P2 verbleibt 13 mg (37%) Methyl-β-D-N-Acetylglucosaminid. Die Verbindung ist nach DC und ¹H-NMR einheitlich
¹H-NMR (D₂O):
δ = 1.88 (s, 3H, CH₃), 3.34 (s, 3H, CH₃O), 4.25 (d, J = 8 Hz, 1H, β-H1)

### Beispiel 3:

50 mg (0.15 mmol) p-Nitrophenyl-β-D-N-Acetylglucosaminid und 2 mL Methanol werden mit 1 Unit N-Acetylglucosaminidase (gereinigtes Enzym) aus T. thermophila in 10 ml 0.1M Citrat-Puffer pH 6.5 19h bei Raumtemperatur gerührt. Nach Extraktion mit Diethylether wird die wäßrige Phase gefriergetrocknet. Nach Chromatographie an Biogel P2® verbleiben 22 mg (62%) Methyl-β-D-N-Acetylglucosaminid. Die Verbindung ist nach DC und ¹H-NMR einheitlich
¹H-NMR (D₂O):
δ = 1.88 (s, 3H, CH₃), 3.34 (s, 3H, CH₃O), 4.25 (d, J = 8 Hz, 1H, b-H1)

### Beispiel 4:

50 mg (0.15 mmol) p-Nitrophenyl-β-D-N-Acetylgalactosaminid und 2 mL Methanol werden mit 1 Unit β-Hexosaminidase (Rohenzym) aus T. thermophila in 10 ml 0.1M Citrat-Puffer pH 6.5 19h bei Raumtemperatur gerührt. Nach Extraktion mit Diethylether wird die wäßrige Phase gefriergetrocknet. Nach Chromatographie an Biogel P2® verbleiben 10 mg (28%) Methyl-β-D-N-Acetylglucosaminid. Die Verbindung ist nach DC und ¹H-NMR einheitlich
¹H-NMR (D₂O):
δ = 1.91 (s, 3H, CH₃), 3.4 (s, 3H, CH₃O), 4.26 (d, J = 8 Hz, 1H, b-H1)

### Beispiel 5:

50 mg (0.15 mmol) p-Nitrophenyl-β-D-N-Acetylgalactosaminid und 2 mL Methanol werden mit 1 Unit N-Acetylglucosaminidase (gereinigtes Enzym) aus T. thermophila in 10 ml 0.1M Citrat-Puffer pH 6.5 48h bei Raumtemperatur gerührt. Nach Extraktion mit Diethylether wird die wäßrige Phase gefriergetrocknet. Nach Chromatographie an Biogel® konnte kein Produkt isoliert werden. Das Enzym akzeptiert p-Nitrophenyl-β-D-N-Acetylgalactosaminid nicht als Substrat. Es handelt sich also um eine reine N-Acetylglucosaminidase.

### Beispiel 6:

100 mg (0.29 mmol) p-Nitrophenyl-β-D-N-Acetylglucosaminid und 200 mg Glucose werden mit 2,1 Unit N-Acetylglucosaminidase aus T. thermophila in 15 ml 0.1M Citrat-Puffer pH 6.5 48h bei Raumtemperatur gerührt. Nach Extraktion mit Diethylether wird die wäßrige Phase gefriergetrocknet. Nach Chromatographie an Biogel P2® verbleiben 92 mg (83%) β-D-GlcNAc-(1→4)-Glc (4-O-β-D-2-Acetamino-2-deoxyglucopyranosyl-D-glucose). Die Verbindung ist nach DC und ¹H-NMR einheitlich
¹H-NMR (D₂O):
δ = 2.05 (s, 3H, CH₃), 3.2-3.9 (m, 12H, H-GlcNAc-Glc), 4.65 (d, J = 8 Hz, 1H, b-H1- GlcNAc), 4.66 (d,J = 8 Hz, b-H1-Glc), 5.19 (d, J = 3 Hz, a-H1-Glc)

### Beispiel 7:

100 mg (0.29 mmol) p-Nitrophenyl-β-D-N-Acetylglucosaminid und 200 mg N-Acetylglucosamin werden mit 2,1 Unit N-Acetylglucosaminidase aus T. thermophila in 15 ml 0.1M Citrat-Puffer pH 6.5 48h bei Raumtemperatur gerührt. Nach Extraktion mit Diethylether wird die wäßrige Phase gefriergetrocknet. Nach Chromatographie an Biogel P2® verbleiben 61 mg (49%) β-D-GlcNAc-(1-4)-GlcNAc .( 4-O-β-D-2-Acetamino- 2-deoxyglucopyranosyl-D-2-acetamino-2-deoxyglucose). Die Verbindung ist nach DC und ¹H-NMR einheitlich
¹H-NMR (D₂O):
δ = 2.05 (s, 3H, CH₃), 2.08 (s. 3H, CH₃), 3.2-3.9 (m, 12H, H-GlcNAc-GlcNAc), 4.65 (d, J = 8 Hz, 1H, b-H1- GlcNAc), 4.7 (d,J = 8 Hz, b-H1-GlcNAc), 5.19 (d, J = 3 Hz, a-H1-GlcNAc)

### Beispiel 8:

200 mg (0.58 mmol) p-Nitrophenyl-β-D-N-Acetylglucosaminid und 150 mg (1R,2R)-trans-1,2-Cyclohexandiol werden mit 1,5 Unit N-Acetylglucosaminidase aus T. thermophila in 10 ml 0.1M Citrat-Puffer pH 6.5 48h bei Raumtemperatur gerührt. Nach Extraktion mit Diethylether wird die wäßrige Phase gefriergetrocknet. Nach Chromatographie an Biogel P2® verbleiben 50 mg (28%) (1R,2R)-trans-1,2-Cyclohexandiol-β-D-2-acetamino-2-deoxy-glucopyranosid. Die Verbindung ist nach DC und ¹H-NMR einheitlich
¹H-NMR (D₂O):
δ = 1.0-1.2 (m, 4H, CH₂-Cyclohexandiol), 1.5-1.6 (m, 2H, CH₂-Cyclohexandiol), 1.78-1.86 (m, 2H, CH₂-Cyclohexandiol), 1.9 (s, 3H, CH₃), 3.3-3.4 (m, 2H, CH-Cyclohexandiol), 3.5-3.8 (m, 6H, GlcNAc), 4.6 (d, J = 8 Hz, 1H, b-H1)

### Beispiel 9:

200 mg (0.58 mmol) p-Nitrophenyl-β-D-N-Acetylglucosaminid und 200 mg 6-N-Benzyloxycarbonylamino-1-hexanol werden mit 1,5 Unit N-Acetylglucosaminidase aus T. thermophila in 10 ml 0.1M Citrat-Puffer pH 6.5 48h bei Raumtemperatur gerührt. Nach Extraktion mit Diethylether wird die wäßrige Phase gefriergetrocknet. Nach Chromatographie an Biogel P2® verbleiben 12 mg (5%) GlcNAc-β-O(CH₂)₆NHZ Die Verbindung ist nach DC und ¹H-NMR einheitlich
¹H-NMR (D₂O):
δ = 1.3 (m, 4H, CH₂-Spacer), 1.4-1.5 (m 4H, CH₂-Spacer), 3.2 (t, 2H, CH₂-O), 3.4-4.0 (m, 8H, GlcNAc, CH₂-NHZ), 4.6 (d, J = 8Hz, b-H1), 5.1 (s, 2H, CH₂Ph), 7.2 (m, 5H. Ph)

### Beispiel 10:

200 mg (0.58 mmol) p-Nitrophenyl-β-D-N-Acetylglucosaminid und 200 mg Z-L-Serinmethylester werden mit 1,2 Unit N-Acetylglucosaminidase aus T. thermophila in 10 ml 0.05M Citrat-Puffer pH 6.5 und 2 ml Dimethylsulfoxid 120h bei Raumtemperatur gerührt. Nach Extraktion mit Diethylether wird die wäßrige Phase gefriergetrocknet. Nach Chromatographie an Biogel P2® verbleiben 12 mg (5%) GlcNAc-β-OCH₂CH(NHZ)COOCH₃ Die Verbindung ist nach DC und ¹H-NMR einheitlich
¹H-NMR (D₂O):
δ = 1.96 (s, 1H, CH₃), 3.3-3.8 (m, 8H, GlcNAc, CH2-Serin), 3.8 (s, 3H, CH₃O), 4.0 (t, 1H, CH-Serin), 4.5 (d, J = 8 Hz, b-H1), 5.0 (s, 2H, CH₂Ph), 7.3 (m, 5H, Ph)

### Beispiel 11:

In 1ml Eppendorf-Cups werden jeweils 500 µl β-Galactosidase aus T. thermophila in 50mM HEPES pH 6.8 und 10 mg o-Nitrophenyl-β-D-galactopyranosid mit verschiedenen Acceptoren (100 µl Methanol, 100 µl Ethanol, 20 mg Xylose, 20 mg N-Acetylglucosamin, 20 mg (1R,2R)-trans-1,2-Cyclohexandiol ) 72h bei 27°C inkubiert. Über DC (Laufmittel: 1-Butanol-Aceton-Eisessig-Wasser 35:35:7:23) konnten die entstandenen Galactoside identifiziert werden.

| | |
|---|---|
| Methyl-β-D-galactopyranosid | R_{f} = 0.7 |
| Ethyl-β-D-galactopyranosid | R_{f} = 0.68 |
| (1R,2R)-trans-1,2-Cyclohexandiol-β-D-galactopyranosid. | R_{f} = 0.72 |
| 4-O-β-D-galactopyranosyl-D-xylose | R_{f} = 0.48 |
| 4-O-β-D-galactopyranosyl-D-2-acetamino-2-deoxy-glucose | R_{f} = 0.44 |

### Beispiel 12:

In 1ml Eppendorf-Cups® werden jeweils 1 ml β-Fucosidase aus T. thermophila in 50mM Phosphatpuffer pH 6.5 und 10 mg o-Nitrophenyl-β-D-galactopyranosid mit verschiedenen Acceptoren (100 µl Methanol, 100 µl Ethanol, 4.5 mg (1R,2R)-trans-1,2-Cyclohexandiol ) 40h bei 37°C inkubiert. Über DC (Laufmittel: 1-Butanol-Aceton-Eisessig-Wasser 35:35:7:23) konnten die entstandenen Fucoside identifiziert werden.

| | |
|---|---|
| Methyl-β-D-fucopyranosid | R_{f} = 0.71 |
| Ethyl-β-D-fucopyranosid | R_{f} = 0.67 |
| (1R,2R)-trans-1,2-Cyclohexandiol-β-D-fucopyranosid. | R_{f} = 0.64 |

### Beispiel 13:

200 mg (0.66 mmol) p-Nitrophenyl-α-D-glucosid und1 ml Methanol werden mit 0.33 Units α-Glucosidase aus T. thermophila in 20 ml 0.05M Citrat-Puffer pH 6.5 48h bei Raumtemperatur gerührt. Nach Extraktion mit Diethylether wird die wäßrige Phase gefriergetrocknet. Nach Chromatographie an Biogel P2® verbleibt 113 mg (88%) Methyl-α-D-glucopyranosid. Die Verbindung ist nach DC und ¹H-NMR einheitlich
¹H-NMR (D₂O):
δ = 3.2-3.76 (m, 6H, Glc), 3.6 (s, 3H, CH₃O), 4.67 (d, J = 3Hz, a-H1)

### Beispiel 14:

200 mg (0.66 mmol) p-Nitrophenyl-α-D-glucopyranosid und 1 ml Ethanol werden mit 1400 µl α-Glucosidase (Rohenzym) aus T. thermophila in 20 ml 0.05M Citrat-Puffer pH 5.5 48h bei Raumtemperatur gerührt. Nach Extraktion mit Diethylether wird die wäßrige Phase gefriergetrocknet. Nach Chromatographie an Biogel P2 verbleibt 29 mg (22%) Ethyl-α-D-glucopyranosid. Die Verbindung ist nach DC und ¹H-NMR einheitlich.
¹H-NMR (D₂O):
δ = 1.4 (t, 3H, CH₃), 3.72 (q, 2H, CH₂O), 3.5-4.0 (m, 6H, Glc), 5.1 (d, J = 4Hz, a-H1)

### Beispiel 15:

200 mg (0.66 mmol) p-Nitrophenyl-α-D-mannopyranosid und 1.5 ml 1-Butanol werden mit 1500 µl α-Mannosidase (Rohenzym) aus T. thermophila in 20 ml 0.05M Citrat-Puffer pH 5.5 48h bei Raumtemperatur gerührt. Nach Gefriertrocknung und Chromatographie mit Methanol-Wasser 2:3 an Biogel P2 verbleibt 36 mg (23%) Butyl-α-D-mannopyranosid. Die Verbindung ist nach DC und ¹H-NMR einheitlich.
¹H-NMR (D₂O):
δ = 0.75 (t, 3H, CH₃), 1.2 (m, 2H, CH2), 1.4 (m, 2H, CH2), 3.38 (q, 2H, CH₂O), 3.4-3.8 (m, 6H, Man), 4.7 (d, J = 4Hz, a-H1)

### Beispiel 16:

200 mg (0.66 mmol) p-Nitrophenyl-β-D-Fucopyranosid und 2 ml Ethanol werden mit 750 µl β-Fucosidase (Rohenzym) aus T. thermophila in 20 ml 0.05M Citrat-Puffer pH 5.5 24h bei Raumtemperatur gerührt. Nach Extraktion mit Diethylether wird die wäßrige Phase gefriergetrocknet. Nach Chromatographie an Biogel P2 verbleibt 48 mg (35%) Ethyl-β-D-Fucopyranosid. Die Verbindung ist nach DC und ¹H-NMR einheitlich.
¹H-NMR (D₂O):
δ = 1.06 (t, 3H, CH₃), 1.1 (d, 3H, CH3(, 3.3 (dd, H2), 3.45-3.84 (m, 6H, Fuc, CH₂O), 4.22 (d, J = 8Hz, b-H1)

## Patentansprüche

1. Verfahren zur Herstellung von Gycosiden durch enzymatische Glycosidierung von Glycosylakzeptoren mittels Glycosidasen, dadurch gekennzeichnet, daß eine Glycosidase aus holotrichen Ciliaten eingesetzt wird.

2. Verfahren nach Anspruch 1, dadurch gekennzeichnet, daß eine Glycosidase aus Hymenostomatiden eingesetzt wird.

3. Verfahren nach Anspruch 2, dadurch gekennzeichnet, daß eine Glycosidase aus Colpidium eingesetzt wird.

4. Verfahren nach Anspruch 2, dadurch gekennzeichnet, daß eine Glycosidase aus Tetrahymena eingesetzt wird.

5. Verfahren nach Anspruch 2, dadurch gekennzeichnet, daß eine Glycosidase aus Pseudocohnilembus eingesetzt wird.

6. Verfahren nach Anspruch 2, dadurch gekennzeichnet, daß eine Glycosidase aus Potomacus eingesetzt wird.

7. Verfahren nach einem der Ansprüche 1 bis 6, dadurch gekennzeichnet, daß als Glycosyldonor Nitrophenylglycoside eingesetzt wird.

8. Verfahren nach einem der Ansprüche 1 bis 6, dadurch gekennzeichnet, daß als Glycosyldonor ein Glycosylfluorid eingesetzt wird.

9. Verfahren nach einem der Ansprüche 1 bis 6, dadurch gekennzeichnet, daß als Glycosyldonor ein Disaccharid eingesetzt wird.

10. Verfahren nach einem der Ansprüche 1 bis 9, dadurch gekennzeichnet, daß als Glycosylakzeptor ein Alkohol mit 1 bis 10 Kohlenstoffatomen eingesetzt wird.

11. Verfahren nach einem der Ansprüche 1 bis 9, dadurch gekennzeichnet, daß als Glycosylakzeptor eine Polyhydroxyverbindung mit 2 bis 10 Kohlenstoffatomen eingesetzt wird.

12. Verfahren nach einem der Ansprüche 1 bis 9, dadurch gekennzeichnet, daß als Glycosylakzeptor ein Monosaccharid eingesetzt wird.

13. Verfahren nach einem der Ansprüche 1 bis 9, dadurch gekennzeichnet, daß als Glycosylakzeptor eine Verbindung der allgemeinen Formel I, 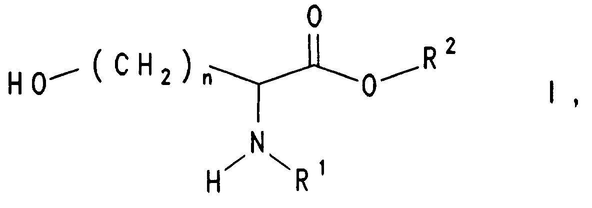 eingesetzt wird, worin bedeuten
n eine ganze Zahl von 1 bis 10,
R¹ eine Aminoschutzgruppe,
R² eine Hydroxylgruppe, eine Alkoxyalkylgruppe, Thioalkylgruppe oder eine Alkenyloxygruppe mit jeweils 1 bis 18 Kohlenstoffatomen, die mit Halogen oder Cyan substituiert sein können, oder eine Aryloxygruppe mit 6 bis 10 Kohlenstoffatomen, die mit Alkyl-, Alkoxy-, Thioalkyl-, jeweils mit 1 bis 5 C-Atomen, und Nitrogruppen substituiert sein kann.
